# EUROPEAN PATENT APPLICATION

(11) **EP 2 545 974 A1**
(43) Date of publication of application: **16.01.2013**
(21) Application number: 11753259.8
(22) Date of filing: 03.03.2011
(51) Int. Cl.: B01D 11/02, B01F 1/00, B01F 3/04

(54) **EXTRACTION METHOD USING MICROBUBBLES AND EXTRACTING LIQUID**

(30) Priority: 08.03.2010 JP 2010051146
(71) Applicant: LIGARIC CO., LTD., Osaka 565-0805 (JP); Sunstar Engineering Inc., Osaka 569-1195 (JP)
(72) Inventor: TSUJI Hideyasu, Fukuyama-shi Hiroshima 721-0955 (JP); TSUJI Yasuhiro, Fukuyama-shi Hiroshima 721-0955 (JP); OKA Toru, Takatsuki-shi Osaka 569-1195 (JP); MIYAO Haruka, Takatsuki-shi Osaka 569-1195 (JP); LIAUW, Denny, 117684 (SG)
(74) Representative: Berryman, Natalia Grace
(86) International application number: PCT/JP2011/054903
(87) International publication number: WO 2011/111600

(57) **Abstract**

The present invention provides an extraction method that either does not use emulsifiers, organic solvents, and the like or can reduce the amount used of emulsifiers, organic solvents, and the like. In addition, the extraction method of present invention can also efficiently extract various components from various materials to be extracted, and can highly maintain potency and the like of the extracted components, in particular if the components are active agents. Furthermore, the extraction method of present invention has excellent safety. The extraction method of present invention is achieved by bringing materials to be extracted into contact with liquid containing ultra fine bubbles for extraction treatment. The ultra fine bubbles utilized during the extraction process preferably have a mode diameter of 500 nm or smaller and a concentration of 1,000,000 or more per 1 ml of liquid.

## Description

### Technical Field

The present invention relates to an extraction method using ultra fine bubbles and the liquid extract obtained by the extraction method.

### Background Art

Conventionally, in order to extract a desired component from a material to be extracted, for example, an aqueous solvent or an organic solvent has been used to extract a component soluble in each solvent, thereby obtaining the component as liquid extract (extract).

For example, when a plant material is used as the material to be extracted to obtain its extract, in many cases, an extraction solvent including water; organic solvents such as methanol, ethanol, butanol, isopropyl alcohol, hexane, heptane, cyclohexane, ethyl acetate, acetone, and petroleum ether; polyols such as 1,3-butylene glycol, glycerin, and propylene glycol; and oils and mineral oils such as castor oil, olive oil, sunflower seed oil, squalene, and liquid paraffin is singly used or these extraction solvents are appropriately mixed to be used, a raw or dried plant is subjected to or not subjected to treatment such as pulverization and fine cutting and then is subjected to, for example, cold immersion, warm immersion, heating and refluxing, or percolation to extract soluble components.

However, there are some problems in such an extraction method from the viewpoints of potency and quality. For example, a conventional steam distillation needs heating or solvent extraction after the distillation and this may alter obtained components or may leave solvent which may cause irritating smell. From the viewpoint of the production, a solvent is required in a relatively large amount and such a solvent has to be removed, for example. In addition, hydrophilic components cannot be extracted.
By extraction methods using water, the amount of extracted components is small and extractable components are limited. On this account, an extraction method using a mixture with a hydrophilic solvent (Patent Document 1) and a method by adding an emulsifier in a small amount (Patent Document 2) have been developed. However, such methods may leave a solvent or an additive, and may raise problems such as limited applications of the extract and safety issue. A method of obtaining an extract by supercritical extraction using carbon dioxide gas in a supercritical state (Patent Document 3) has also been developed, but has problems of, for example, the cost and apparatus.
The extraction using animal materials, soils, and ores as materials to be extracted also has substantially the same problems as above.

### Citation List

### Patent Literatures

Patent Document 1: JP-A No. 10-202002
Patent Document 2: JP-A No. 2002-84992
Patent Document 3: JP-A No. 2000-237501

### Summary of Invention

### Technical Problem

In view of the above problems, an object of the present invention is to provide an extraction method that does not use emulsifiers, organic solvents, and the like or can reduce the amount used of emulsifiers, organic solvents, and the like, can efficiently extract various components from various materials to be extracted, can highly maintain potency and the like of an extracted component when the component is an active component, and further has excellent safety; and liquid extract obtained by the extraction method.

### Solution to Problems

The present inventors have carried out intensive studies in order to solve the above problems, as a result, have found that, by using water containing ultra fine bubbles, various components can be efficiently extracted from various materials to be extracted without using emulsifiers, organic solvents, and the like; or using emulsifiers, organic solvents, and the like in reduced amount, and the invention has been accomplished.

The summary of the present invention is as follows.
(1) The present invention relates to an extraction method including bringing materials to be extracted into contact with water containing ultra fine bubbles for extraction treatment.

(2) The present invention relates to the extraction method in which the ultra fine bubbles have a mode diameter of 500 nm or smaller.

(3) The present invention relates to the extraction method in which 1 mL of the water contains 1,000,000 or more of the ultra fine bubbles.

(4) The present invention relates to the extraction method in which the ultra fine bubbles have an electrically charged surface and the surface has an absolute zeta potential of 5 mV or more.

(5) The present invention relates to the extraction method in which the ultra fine bubbles substantially include one or more gases selected from air, oxygen, hydrogen, nitrogen, carbon dioxide, ozone, and inert gases. In the present invention, the inert gases mean noble gases such as helium, neon, and argon.

(6) The present invention relates to the extraction method in which the water contains water-soluble solvents in an amount of 8% by weight or less.

(7) The present invention relates to the extraction method in which the material to be extracted is at least one selected from animal materials, a plant materials, soils, and an ores.

(8) The present invention relates to the extraction method further including applying one or more waves selected from electromagnetic waves, sound waves, and ultrasonic waves as the extraction treatment.

(9) The present invention relates to the extraction method in which the ultra fine bubbles are generated using a ultra fine bubble generating apparatus equipped with a gas-liquid shearing mixer.

(10) The present invention relates to the extraction method in which the materials-to-be extracted are firstly immersed in water, and the ultra fine bubbles are simultaneously generated in this materials-to-be extracted containing liquid by ultra-fine bubbles generating apparatus.

(11) The present invention relates to liquid extract obtained by the extraction method as described in any one of the items (1) to (10).

(12) The present invention relates to an extract powder obtained by drying the liquid extract as describe in the item (11).

(13) The present invention relates to a composition containing the liquid extract as described in the item (11) or the extract powder described in the item (12).

### Benefits of the Invention

According to the extraction method of the present invention, various components can be efficiently extracted from various materials to be extracted without using emulsifiers, organic solvents, and the like; or with emulsifiers, organic solvents, and the like in a reduced amount. The method can highly maintain the potency and the like of an extracted component when the component is an active component. The method may not use emulsifiers, organic solvents, and the like; or may use emulsifiers, organic solvents, and the like in reduced amount, thereby providing an extraction method having excellent safety.

### Description of Embodiments

The extraction method of the present invention is characterized by bringing a material to be extracted into contact with water containing ultra fine bubbles for extraction treatment to extract various components from the material to be extracted. The components can encompass a wide range of components from hydrophilic components to hydrophobic components and the water that has been used in the extraction method of the present invention can simultaneously contain these components that have been extracted from a material to be extracted by the action of the ultra fine bubbles.

In the present invention, the ultra fine bubbles mean bubbles that are so-called nanobubbles, have a maximum width (generally, diameter) of several hundreds of nanometers or smaller, and can be present in water for a very long time. In the present invention, such ultra fine bubbles may be used. From the viewpoint of extraction efficiency, the ultra fine bubbles preferably have a mode diameter of 500 nm or smaller, more preferably 300 nm or smaller, particularly preferably 150 nm or smaller, and most preferably 50 nm or smaller. When components are extracted from, for example, animal or plant materials, it is supposed that extremely fine bubbles having a particle diameter within this range can achieve improved penetration efficiency between cell membranes, thereby improving permeability of the ultra fine bubbles into the materials.

The number of the ultra fine bubbles present in the water containing ultra fine bubbles is not specifically limited. From the viewpoint of the extraction efficiency, the number of the ultra fine bubbles in 1 mL of the water is preferably 1,000,000 or more, more preferably 8,000,000 or more, even more preferably 30,000,000 or more, and most preferably 50,000,000 or more. When components are extracted from, for example, animal or plant materials, it is supposed that the presence of ultra fine bubbles in such a range increases the number of bubbles which are in contact with a hydrophilic part or a hydrophobic part of each extracted component, thereby contributing to the stabilization of each component.

The mode diameter and the number of bubbles are the values obtained by measurement with a nanoparticle analysis system, NanoSight series (manufactured by NanoSight). The nanoparticle analysis system, NanoSight series (manufactured by NanoSight) measures the speed of Brownian motion of nanoparticles and calculates the particle size and the particle number based on the speed. The system can also determine the size and the number of bubbles in a similar manner. The concentration and mode diameter of the bubbles can be ascertained by the particle size distribution of nanoparticles measured.

In the present invention, the ultra fine bubbles preferably have an electrically charged surface and the surface preferably has an absolute zeta potential of 5 mV or more and more preferably 7 mV or more. It is supposed that ultra fine bubbles having a surface with a zeta potential within such a range enhance the stabilization of an extraction component due to a surface activation effect derived from the zeta potential on the bubble surface. In particular, when components are extracted from animal or plant materials, surface hydrophobicity of ultra fine bubbles increases the permeability efficiency into cell membranes as well as can stabilize the hydrophobic part of an extraction component.
The zeta potential is used as an index for evaluating dispersion stability, agglutinating property, interaction, and surface modification of colloidal particles and it is widely known that a larger absolute value shows more stable dispersibility. This is because colloidal particles are electrically charged and the electrostatic repulsion between the particles corresponds to the zeta potential. Accordingly, a higher absolute value further increases the colloidal stability and thus ultra fine bubbles can be stably present. Therefore, it is supposed that a higher absolute value further increases the surface activation effect, thereby providing a preferred condition for the extraction of various components.

The ultra fine bubbles may be generated in water using gas, substantially including one or more gases selected from air, oxygen, hydrogen, nitrogen, a carbon dioxide gas, ozone, and inert gases. Typically, air is preferably used. A gas having no reactivity with a component to be extracted or a gas having a reactivity that is not practically considered may be used. Conversely, a gas having a reactivity may be used for positive modification of an extraction component. Here, the term "substantially including" means that other components may be included in a trace amount in addition to the components above, and the inert gases means a noble gas such as helium, neon, and argon as described above.

The ultra fine bubbles as above can be generated by an arbitrary known means, for example, a static mixer system, a Venturi system, a cavitation system, a steam condensate system, an ultrasonic system, a swirling flow system, a pressurized dissolution system, and a microporous system. A preferred bubble generating system is a gas-liquid shearing mixing system.

A useful apparatus using the gas-liquid shearing mixing system for generating ultra fine bubbles can be exemplified by a ultra fine bubble generating apparatus disclosed in Japanese Patent No. 4118939. In the apparatus, most of a gas-liquid mixed fluid introduced into a fluid swirling chamber does not simply move to an outlet as in a conventional apparatus but once moves in the opposite direction from the outlet as a swirling flow. Then, the swirling flow is reversed by a first end wall member to move from the first end wall member toward a second end wall member. At this time, the radius of rotation of the swirling flow is smaller than that of the swirling flow moving toward the first end wall member. Therefore, the flow rate is increased to increase the shear force acting on the gas contained in the gas-liquid mixed fluid and breaking up of the gas is accelerated.

Examples of the water used in the present invention include various waters such as distilled water, ultrapure water, highly pure water, pure water, tap water, ion-exchanged water, filtered water, and natural water.

The water may contain water-soluble solvents in a small amount as a co-solvent if the solvents have no influence to the extracted materials
In the present invention, the water may contain the water-soluble solvent in an amount of 8% by weight or less and preferably 5% by weight or less. The use of a water-soluble solvent in a small amount in this manner can improve the extraction efficiency depending on the components to be extracted and also the combination use with ultra fine bubbles enables the reduction of the amount of the water-soluble solvent used comparing with using water
The water-soluble solvents may be appropriately selected in consideration of safety and the like depending on an application. Examples of the water-soluble solvents include lower monohydric alcohols (such as methanol, ethanol, 1-propanol, 2-propanol, 1-butanol, and 2-butanol), polyhydric alcohols (such as 1,3-butylene glycol and propylene glycol), ketones (such as acetone and methyl ethyl ketone), and acetonitrile. For example, from the viewpoint of safety, ethanol, acetone, and the like are preferred.

The material to be extracted is not specifically limited and various materials can be used. For example, at least one material selected from animal materials, plant materials, a soils, oil sand, and ores may be used. The material to be extracted, such as animal materials or plant materials may be used as a raw material or as a dried material for extraction treatment. Alternatively, in order to further increase the extraction efficiency, such a material may be subjected to pretreatment such as pulverization, fine cutting, freezing, heating, powderizing, chipping, immersion in solvents, pressurization with gas, and irradiation with a radiation ray.

Examples of the animal materials usable include, but are not necessarily limited to, experimental and livestock animals, fishes and shellfishes, and other various animals.
Examples of the expected extraction components include, but are not necessarily limited to, components derived from animals described in the book "Corpus of Animal Component Utilization, Land Animals" (published by NTS, June 2002) and components derived from marine animals, snakes, insects, animals used for Kampo medicines, and the like described in the book "Corpus of Animal Component Utilization, Marine Animals, Snakes, Insects, and Kampo medicines" (published by R & D Planning, December 1986). More specifically, examples include peptides, proteins, glycoproteins, glycolipids, phospholipids, carbohydrates, fatty acids, hormone-like substances, amine substances (such as catecholamine, acetylcholine, serotonin, and histamine), amino acid substances (such as glutamic acid, aspartic acid, gamma-amino acid, glycine, taurine, and serine), nucleic acid substances (such as inosinic acid), adenosine, ATP, angiotensin II, mucopolysaccharides (glycosaminoglycan, keratan sulfate, hyaluronic acid, and chondroitin sulfate), enzymes (trypsin, chymotrypsin, amylase, lipase, and protease), and minerals (such as calcium, zinc, sodium, and potassium).

Examples of the plant materials usable include, but are not necessarily limited to, trees (such as tree barks, berries (fruits), leaves, and roots), wild plants, vegetables, mushrooms, marine algae, and other plant materials.
Examples of the expected extraction components include, but are not necessarily limited to, components described in the book "Synthetic Perfume" (published by The Chemical Daily Co., Ltd, February 2005), such as hydrocarbons, alcohols, phenols and derivatives of the phenols, aldehydes, acetals, ketones, ketals, ethers, oxides, synthetic musks, acids, lactones, esters, nitrogen-containing compounds, sulfur-containing compounds, and halogen-containing compounds.
More specific examples are as below.
Examples of the terpenes include azadirachtin A, azadirachtin B, nimbin, salannin, nimbidin, meliantriol, glycyrrhizin, citral, taxol, α-pinene, 1-menthol, camphor, and ginkgolide.
Examples of the flavonoids include chalcones (carthamin), flavanones (naringenin, naringin (glycoside), hesperidin, citronetin, citronin (glycoside), liquiritigenin, and liquiritin), flavones (apigenin, chrysin, luteolin, apiin, baicalin, baicalein, wogonin, and tricin), flavonols (galangin, quercetin, rutin (glycoside), quercitrin, kaempferol, myricetin, and fisetin), flavanols (alpinone), flavanols (catechins) (catechin, epicatechin, epigallocatechin, epigallocatechin gallate, epicatechin gallate, theaflavin, and thearubigin), isoflavones (daidzein, daidzin (glycoside), genistein, genistin, and puerarin), anthocyanidins (anthocyanidin, cyanidin, cyanine (glycoside), pelargonidin, pelargonin (glycoside), peonidin, petunidin, and malvidin), and stilbenoids (resveratrol).
Examples of the phenolic acids include lignans (sesamin, sesamolin, sesaminol, and sesamol), caffeic acid, ferulic acid, rosmarinic acid, and chlorogenic acid (neochlorogenic acid, and cryptochlorogenic acid).
Examples of the alkaloids include aconitine, atropine, arecoline, scopolamine, theobromine, and caffeine.
Examples of the nucleic acids include guanylic acid.
Additional examples of the nitrogen-containing compounds include coffee melanoidins.
The above components are specific examples and the invention is not limited to them.

With regard to the soils and the oil sands, any materials may be used as extraction materials without particular limitation, for example, extraction of microorganisms and oils contained in soils and oil sands, heavy metals contained in contaminated soils, and harmful components such as environmental hormones
Specific examples of the soil contaminant include, but are not limited to, lead, cadmium, chromium, tin, total cyanide, hexavalent chromium, organic phosphorus, arsenic, total mercury, alkyl mercury, PCB, copper, dichloromethane, carbon tetrachloride, 1,2-dichloroethane, 1,1-dichloroethylene, cis-1,2-dichloroethylene, 1,1,1-trichloroethane, 1,1,2-trichloroethane, trichloroethylene, tetrachloroethylene, 1,3-dichloropropene, thiuram, simazine, thiobencarb, benzene, selenium, fluorine, boron, dioxin, bisphenol A, and tributyltin.

For the ores, any materials may be used as extraction materials without particular limitation, for example, various minerals and rare metals that are highly rare and industrially important, contained in ores.
Specifically expected extraction components are, for example, nickel, chromium, manganese, cobalt, tungsten, molybdenum, vanadium, niobium, tantalum, germanium, strontium, antimony, platinum group elements, ilmenite, rutile, beryllium, zirconium, rhenium, lithium, boron, gallium, barium, selenium, tellurium, bismuth, indium, cesium, rubidium, thallium, hafnium, and rare earth elements (scandium, yttrium, lanthanum, cerium, praseodymium, neodymium, promethium, samarium, europium, gadolinium, terbium, dysprosium, holmium, erbium, thulium, ytterbium, and lutetium).

The form of the materials to be extracted is not specifically limited. However, in order to improve the extraction efficiency by maximizing the contact area between ultra fine bubbles and materials to be extracted, as necessary, the materials to be extracted is preferably subjected to, for example, pulverization to reduce the particle size prior to the extraction process.

The extraction method of the present invention is carried out by bringing the material to be extracted into contact with the water containing ultra fine bubbles. The contact method is not specifically limited and various methods such as immersion and spraying may be employed. The material may be brought into contact either in a batch system or in a continuous system. The conditions such as contact temperature and contact time may be appropriately determined depending on, for example, the contact method, characteristics of the material to be extracted, and characteristics of the extraction components. For example, in the case that a material to be extracted is brought into contact with the ultra fine bubbles containing water by immersion in a batch system, the contact ratio (ultra fine bubbles containing water/material to be extracted, mixing ratio by weight) of the ultra fine bubbles containing water with respect to the material to be extracted is about 0.1 to 100,000, more preferably 0.1 to 50,000, even more preferably 0.1 to 20,000, and most preferably 0.1 to 5,000, the contact temperature is about 4 to 100°C, preferably 10 to 60°C, and more preferably 15 to 35°C, and the contact time is 1 to 180 minutes. However, these conditions may be appropriately determined depending on the material to be extracted and the extraction components. The contact temperature and the extraction time may be substantially the same as the temperature and the time described above, but are not limited to them. The extraction may be carried out at elevated pressure and temperature depending on the types of the material to be extracted and extraction components. When the ultra fine bubbles are used in this manner, in particular, components that may be altered by heat can be extracted without conventional high temperature treatment, thereby suppressing the alteration of the extraction components.

For more efficient extraction treatment, stirring or wave irradiation may be combined, and such a treatment condition may be appropriately determined depending on characteristics of a material to be extracted and characteristics of the extraction components. Examples of the wave irradiation treatment include, but are not necessarily limited to, sound wave irradiation, ultrasonic wave irradiation, and electromagnetic wave (such as light wave, heat (infrared light), and microwave) irradiation.

For example, in the case that a material to be extracted is brought into contact with the water by immersion in a batch system, the stirring rotation speed is about 0 to 450 rpm for the stirring condition. When ultrasonic treatment is performed as a wave irradiation treatment, the ultrasonic wave preferably has a frequency of about 20 to 100 kHz as the extraction condition but is not limited to the range.

In the present invention, the water containing ultra fine bubbles may be previously prepared and a material to be extracted may be brought into contact with the ultra fine bubbles containing water; or a material to be extracted may be mixed with water in advanced, and then the ultra fine bubbles are generated in the mixtures. The latter manner is typically effective when a material to be extracted is brought into contact by immersion.

In the present invention, the ultra fine bubbles may be generated by various systems as described above, but are preferably generated using a ultra fine bubbles generating apparatus equipped with a gas-liquid shearing mixer as a gas-liquid shearing mixing system. This is because the apparatus can easily generate the ultra fine bubbles having a mode diameter of 500 nm or smaller in the water or a liquid containing water in which a material to be extracted is immersed.

After the extraction treatment with the water containing ultra fine bubbles as above, extraction residue is removed to obtain liquid extract (also called extract). Examples of the method for removing the residue include known methods such as filtration, centrifugation, filter press, and distillation. Alternatively, after the extraction treatment, the residue is not removed and a mixture of the extraction residue and the extracting liquid may be used without treatment.

The liquid extract obtained as above is a water containing various components extracted from the material to be extracted in a stabilized condition by the ultra fine bubbles. The various components can encompass hydrophilic components as well as components with low water-solubility (hydrophobic components). When the material to be extracted is an animal or plant material, extraction components are commonly known as active agents and can be used, for example, for various pharmaceutical products, vitamins, cosmetics, perfumes, deodorizers, insecticides, bactericides, anti-allergic agents, agrochemicals, fertilizers, seasonings, beverages, and foods. When the material to be extracted is a soil, for example, the extraction of contaminants with low water-solubility or hydrophobicity enables easy purification of the soil, and microorganisms in soil and the like can be collected. When the material to be extracted is an ore, extraction components are commonly supposed to be scarce metals and rare metals and can be used, for example, in various industries such as a home electric appliances industry, an IT industry, and an automobile industry as, for example, structural materials, electronic materials, magnetic materials, and a functional materials.

For the separation of various components extracted as above, the ultra fine bubbles may be destroyed to eliminate the stabilization effect on each component due to the ultra fine bubbles, thereby separating an aqueous phase from a nonaqueous phase, and a desired component alone may be isolated from each phase by a known method.

Hereinafter, as an example in which the material to be extracted is an animal or plant material, embodiments of the liquid extract, the extract powder, and a composition containing the liquid extract or the extract powder will be described.

The liquid extract obtained as above using an animal or plant material as the material to be extracted may be used for the various applications above without treatment or may be diluted or concentrated in a common procedure.
The liquid extract may be dried to form a powder extract. The drying method is not specifically limited, and common methods such as spray drying and freeze drying may be adopted. In order to suppress the decomposition of active components due to heat, the freeze drying is preferably adopted.
To the liquid extract, various additives may be appropriately added depending on the various applications above and forms, as necessary, to afford a composition. The additives usable in the present invention is not specifically limited as long as a function of the extraction component is not impaired, and examples of the additives include, but are not necessarily limited to, water-soluble solvents, water-soluble solids, thickeners, gelling agents, perfumes, moisturizers, excipients, antifungal agents, antiseptics, pharmaceutical drugs, sweeteners, and spices.
The composition may be in various forms such as a liquid form, a cream form, a gel form, a square form, and a powder form. For example, a gel-like composition can be obtained by adding compounds such as agar, carrageenan, gelatin, water absorbent resins, and aqueous polymers into distilled water, warming the mixture to prepare a solution containing the compound, then stirring and mixing the solution with the liquid extract, and cooling the mixture to room temperature. Such a gel-like composition may be used as mist using an atomizer. While, for example, a powder composition can be obtained by appropriately adding, for example, a water-soluble solid such as sugars, polyols, amino acids, and inorganic or organic salts to the liquid extract, as necessary, mixing the mixture to prepare a liquid composition, and drying the liquid composition by a method such as spray drying and freeze drying.
The liquid extract, the extract powder, and the composition containing the liquid extract or the extract powder obtained according to the present invention can be obtained using an animal or natural plant material as the material to be extracted and by merely bringing such a material into contact with the water containing ultra fine bubbles. Therefore, foods, beverages, neutralizers of allergy causing substances, and the like with excellent safety can be provided.

The material to be extracted and the extraction components will be specifically described with reference to specific examples. Neem kernels, as a material to be extracted, is known to contain azadirachtins A and B, nimbin, and salannin as active components. These active components are considered to have inactivation effect of dead mites and the like (allergy causing substances), insect pest repellent action, bactericidal activity, and various pharmaceutical effects, and such effects can be exerted by the extraction components. Liquid extracts containing such active components as the extraction components and a composition containing the liquid extract are suitable for, for example, inactivator of allergy-causing substances (allergen), pest repellent, insect repellent, and bactericide.
For example, the liquid extract obtained by removing an extraction residue as above is immediately subjected to, for example, dilution, concentration, or sterilization, as necessary, to produce a liquid extract that can be used as, for example, an insect repellant or an allergen inactivator without further treatment.
To the liquid extract, for example, water-soluble solvents such as ethanol, antifungal agents, or perfumes might be appropriately added in a required amount to produce a composition that can be similarly used as, for example, an insect repellant or an allergen inactivator.
The addition of, for example, capsaicin as an additive having high insect pest repellent effect to the extraction component can produces an insect pest repellent having higher effect due to a synergistic effect.
Such an allergen inactivator, an insect repellant, or an pest repellent is preferably used in a liquid form to be sprayed using an atomizer (for example, a spray). In the case of an allergen inactivator, such an agent is preferably applied by spray-coating to, for example, indoor spaces, clothes, a carpets, and blankets. In the case of an insect repellant, such an agent may be applied by spray-coating to, for example, skins, window screens, and clothes. In the case of an pest repellent, such an agent may be used by direct spraying, for example, to indoor spaces, to closets, walls, sinks, around pipe lines, basements, attics, and trash boxes in indoor environment, and to flowering plants, soils, and vegetables in outdoor environment.

Furthermore, neem tree bark is known to include tannins as active components. The tannins (tannic acids) derived from the neem tree bark are considered to have pharmaceutical effects and the like, such as inactivation effect of dead mites and the like (allergy causing substances). Hence, the liquid extract obtained using the neem tree bark as the material to be extracted is expected to include the tannins (tannic acids) as the extraction components. In a similar manner to azadirachtins derived from the neem seeds, the liquid extract and a composition containing the liquid extract can be used as an insect repellant and an pest repellent.

In addition, neem leaves may be used as the material to be extracted. The active components have not been specified but the leaves include quercetin, tannic acids, and rutin. The extraction component of neem leaves is considered to have a beneficial effect on skin diseases (for example, acne and rash). Accordingly, to the liquid extract (as necessary, concentrated) derived from the neem leaves, additives such as surfactants, antiseptics, antioxidants, stabilizers, antifreezing agents, buffers, chelating agents, animal or plant oils, waxes, higher alcohols, silicones, water-soluble polymers, polyhydric alcohols, animal or plant extracts, coloring agents, pharmaceutically effective components, pH adjusters, and perfumes may be appropriately added to produce a composition that can be used as cosmetics in a cream form, a gel form, a foam form, an emulsion form, a liquid form, a paste form, a powder form, a granular form, or a solid form (for example, a facial cleansing agent, a facial mask, a beauty essence, a cream, an emulsion, and a skin lotion).

When dried bonito flakes, kombu kelp, and shiitake mushrooms are used as the materials to be extracted, inosinic acid, glutamic acid, and guanylic acid are expected as the extraction components, respectively. These components are widely known as umami components. Accordingly, the liquid extract obtained by using such a material to be extracted is subjected to, for example, dilution, concentration, or sterilization, as necessary, to be used as an edible soup stock (dashi) without further treatment. Furthermore, to the liquid extract, other seasonings may be added to afford a composition that can be used as an edible sauce. The edible sauce may be concentrated in procedures known of those of skill in the art to give a concentrated edible sauce.
The liquid extract or the composition can be dried by, for example, spray drying or freeze drying to afford powdery (for example, powder form and granular form) seasonings (for example, instant soup stocks).

Coffee beans as the materials to be extracted are known to include, for example, chlorogenic acid (neochlorogenic acid, cryptochlorogenic acid), caffeine, and coffee melanoidin, and these components are expected as the extraction components. For example, in recent years, chlorogenic acid has been drawing attention as substance that can inhibits free radical generation, suppresses blood glucose level, enhances fat-burning, suppresses cancer metastasis, and as an antioxidant substance. Caffeine is considered to have a fat decomposition enhancement activity. Accordingly, the liquid extract is expected to include these components as the extraction components and a composition containing the liquid extract are suitable for, for example, foods and beverages (for example, health foods, health beverages, diet foods, and diet beverages) having such activity. Such liquid extract and the composition may also be used as slimming cosmetics (for example, a body wrap, a body scrub, and a body svelte cream) to promote the fat-burning activity
For the shape of the liquid extract and a composition containing the liquid extract, in the case of beverages, liquid extract may be subjected to, for example, dilution, concentration, and sterilization, as necessary, to afford beverages without further treatment. As necessary, for example, sweeteners may be appropriately added to provide a liquid composition as a beverage. In the case of foods, to the liquid extract, for example, gelling agents (such as agar and gelatin) and sweeteners may be appropriately added to afford jelly foods.
The liquid extract may be processed into powders by drying such as spray drying and freeze drying. The extract powders obtained in this manner may be eaten without further treatment, may be added to, for example, water (warm water) or milk beverages to afford beverages, or may be added to various foods.
For using the extraction components as slimming cosmetics, for example, but not limited to, lipophilic components containing essential oils and surfactants and aqueous component containing the liquid extract may be stirred and mixed to afford an emulsified composition, or the extract powder may be added to commercially available slimming cosmetics to afford a composition.

In particular, chlorogenic acid is known to be easily affected by heat. In order to extract chlorogenic acid in larger amounts, unroasted coffee beans may be used as the coffee beans.

When leaves of Perilla frutescens are used as the material to be extracted, expected extraction components of the leaves are for example, rosmarinic acid, ferulic acid, and caffeic acid. Rosmarinic acid has been drawing attention as a substance having functions such as an antimicrobial activity, an antioxidant activity, an anti-allergic activity, a blood glucose level suppression activity, and a sedative activity (relaxing effect); ferulic acid has been drawing attention as a substance having functions such as an antioxidant activity, a free radical generation inhibition activity, and a cancer metastasis suppression activity; and caffeic acid has been drawing attention as a substance having functions such as an anticancer activity. Accordingly, the liquid extract is expected to include these components as the extraction components and a composition containing the liquid extract are suitable for, for example, foods and beverages (for example, health foods, health beverages, diet foods, and diet beverages) having such an activity.
For the shape of the liquid extract and a composition containing the liquid extract, in the case of beverages, the liquid extract may be subjected to, for example, dilution, concentration, and sterilization, as necessary, to afford beverages without further treatment. As necessary, for example, sweeteners may be appropriately added to provide a liquid composition as beverages. In the case of foods, to the liquid extract, for example, gelling agents (such as agar and gelatin) and sweeteners may be appropriately added to afford jelly foods.
The liquid extract may be further processed into powders by drying such as spray drying and freeze drying. The extract powders obtained in this manner may be eaten without further treatment, may be added to, for example, water (warm water) or carbonated beverage to afford beverages, or may be added to various foods. The extract powders may also be mixed with excipients to be processed into tablets in procedures known to those of skills in the art

The type of Perilla frutescens may be either red perilla or green perilla, but in order to extract rosmarinic acid in larger amounts, red perilla is preferred.

When rosemary is used as the material to be extracted, an expected extraction component from the material is, for example, rosmarinic acid. As described above, rosmarinic acid has been drawing attention as a substance having functions such as antimicrobial activity, antioxidant activity, anti-allergic activity, blood glucose level suppression activity, and sedative activity (relaxing effect). Accordingly, the liquid extract is expected to include this components as the extraction component and a composition containing the liquid extract is suitable for, for example, foods and beverages (for example, health foods, health beverages, diet foods, and diet beverages) having such activity. By utilizing, especially, sedative activity (relaxing effect), the liquid extract may also be used as aromatherapy agent or air freshener for home use.
In the case of foods and beverages, the shapes of the liquid extract and a composition containing the liquid extract are the same as the cases of the liquid extract and the composition from leaves of Perilla frutescens. In the case of air freshener, in a similar manner to the above, the extracting liquid may be subjected to, for example, dilution, concentration, and sterilization, as necessary, to be used without further treatment, or to the liquid extract, for example, water-soluble solvents such as ethanol, antifungal agents, and perfumes may be appropriately added in a required amount to afford a liquid composition. Such a composition can be used as air spray or atomizing agent for spraying the air freshener to indoor space. To such a liquid composition, for example, gelling agents, antifungal agents, and perfumes may be appropriately added in a required amount to afford a composition in gel form. Such a gel composition is placed in a room to disperse the air freshener, thereby providing the effect.

It is considered that capers, apples, teas (tea leaves), onions, grapes, broccolis, mulukhiyas, raspberries, bilberries, cranberries, Opuntias, other leafy vegetables, citrus fruits, and the like contain quercetin which is classified into flavonols as an active component; Allium tuberosum, broccoli, daikons, onions, grapefruits, propolis, and the like contain kaempferol which is classified into flavonols as an active component; various vegetables and fruits contain fisetin which is classified into flavonols as an active component; celeries, parsleys, bell peppers, and the like contain luteolin which is classified into flavones as an active component; lemon peel and juice, orange peel, and the like like contain hesperidin which is classified into flavanones as an active component; and pomelo peel and the like contain naringenin which is classified into flavanones as an active component.
These active components are known as polyphenols. The polyphenols are generally considered to have an allergen inactivation activity. Accordingly, when the vegetables and fruits are used as the materials to be extracted, the liquid extract is expected to include the various polyphenols as the extraction components.
Therefore, for example, the liquid extract is obtained by removing an extraction residue as above is immediately subjected to, for example, dilution, concentration, or sterilization, as necessary, to afford liquid extract that can be used as, for example, an allergen inactivator without further treatment.
To the liquid extract, for example, water-soluble solvents such as ethanol, antifungal agents, or perfumes are appropriately added in a required amount to afford a composition that can be similarly used as, for example, an allergen inactivator. Such an allergen inactivator may be in a liquid form. The liquid extract and the liquid composition may be sprayed using an atomizer (for example, a spray) to, for example, indoor spaces, clothes, carpets, and blankets.

### Examples

The present invention will be specifically described hereinafter with reference to examples.

### (Preparation Example 1)

Using a ultra fine bubbles generating apparatus (BUVITAS, manufactured by Kyowakisetsu Co., Ltd.) with a gas-liquid shearing mixing system, ultra fine bubbles containing air were generated in ion-exchanged water to prepare water containing ultra fine bubbles. In the water, the ultra fine bubbles had a mode diameter of 100 nm and a zeta potential of -20 mV and the total number of ultra fine bubbles having a size of 500 nm or smaller was 0.8 × 10⁸/mL. The zeta potential was determined with a zeta potential analyzing system ELSZ-1 manufactured by Otsuka Electronics Co., Ltd.

### (Example 1)

Neem kernels (Nature neem) were pulverized with a mixer, and to 1 g of the obtained pulverized product, 20 mL of the ion-exchanged water containing ultra fine bubbles obtained in Preparation Example 1 was added to prepare a mixture. The mixture was sonicated at 40 kHz with an ultrasonic apparatus (US CLEANER, type USD-4R, manufactured by As One Corporation) at 21°C for 15 minutes to perform extraction treatment. Then, a residue was removed by filtration with a filter paper to afford a filtrate (also called liquid extract). The obtained liquid extract was analyzed by high performance liquid chromatography (Lachrom Elite, manufactured by Hitachi High-Technologies Corporation) to determine the amounts of azadirachtins A and B, nimbin, and salannin contained in the liquid extract. The measurement results are shown in Table 1.
The measurement condition was as follows. Water was used as a solvent A and acetonitrile was used as a solvent B in a gradient condition of 30-40% of the solvent B from 0 to 10 minutes, 40-45% of the solvent B from 10 to 15 minutes, 15-50% of the solvent B from 15 to 20 minutes, 50-60% of the solvent B from 20 to 25 minutes, and 60-70% of the solvent B from 25 to 35 minutes. The flow rate was 1.0 mL/min, the detection was at 217 nm, and a C 18 column was used.

### (Example 2)

The extraction treatment, the filtration, and the analysis of the filtrate (liquid extract) were carried out in the same manner as in Example 1 except that the mixture was shaken (rotation speed: 100 rpm) with a shaking apparatus (Shaker SA300, manufactured by Yamato) for 60 minutes in place of the sonication and the extraction temperature was room temperature (18 to 25°C). The measurement results are shown in Table 1.

### (Example 3)

The extraction treatment, the filtration, and the analysis of the filtrate (liquid extract) were carried out in the same manner as in Example 2 except that 20 mL of a solvent containing-water in which ethanol (EtOH) was added to the ion-exchanged water containing ultra fine bubbles of Preparation Example 1 so as to have a concentration of 2% by weight. The measurement results are shown in Table 1.

### (Comparative Example 1)

The extraction treatment, the filtration, and the analysis of the filtrate (liquid extract) were carried out in the same manner as in Example 1 except that 20 mL of ion-exchanged water without ultra fine bubbles was used in place of the water containing ultra fine bubbles. The measurement results are shown in Table 1.

### (Comparative Example 2)

The extraction treatment, the filtration, and the analysis of the filtrate (liquid extract) were carried out in the same manner as in Example 2 except that 20 mL of ion-exchanged water without ultra fine bubbles was used in place of the water containing ultra fine bubbles. The measurement results are shown in Table 1.

### (Comparative Example 3)

The extraction treatment, the filtration, and the analysis of the filtrate (liquid extract) were carried out in the same manner as in Example 3 except that 20 mL of a solvent containing-water containing 10% by weight of ethanol (EtOH) was used in place of the water containing ultra fine bubbles. The measurement results are shown in Table 1.

**[Table 1]**

| | | | Example 1 | Example 2 | Example 3 | Comparative Example 1 | Comparative Example 2 | Comparative Example 3 |
|---|---|---|---|---|---|---|---|---|
| Extraction condition | Extraction time | [min] | 15 | 60 | 60 | 15 | 60 | 60 |
| | Microbubbles | - | With | With | With | Without | Without | Without |
| | Ultrasound frequency | [kHz] | 40 | - | - | 40 | - | - |
| | Shaking | - | Without | With | With | Without | With | With |
| | EtOH concentration | [% by weight] | 0 | 0 | 2 | 0 | 0 | 10 |
| | Extraction temperature | [°C] | 21 | Room temperature | Room temperature | 21 | Room temperature | Room temperature |
| Extraction amount | Azadirachtin A | [µg/mL] | 24.13 | 3796 | 38.65 | 9 44 | 25 55 | 38 99 |
| | Azadirachtin B | | 1201 | 21 76 | 23.11 | 524 | 1426 | 22 32 |
| | Nimbin | [µg/mL] | 0.73 | 0 96 | 1 4 | 0.24 | 066 | 1 88 |
| | Salannin | [µg/mL] | 1.26 | 1 48 | 2.56 | 0 45 | 0.8 | 3.56 |

As shown in Table 1, the comparisons between Example 1 and Comparative Example 1 and between Example 2 and Comparative Example 2 reveal that the extraction treatment with the water containing ultra fine bubbles increased the extraction amounts. The comparison between Example 2 and Comparative Example 3 reveals that the extraction amounts of azadirachtin A and azadirachtin B were particularly substantially the same. The comparison between Example 3 and Comparative Example 3 reveals that even when the ethanol amount was reduced, nimbin and salannin which are hydrophobic components could be extracted and the extraction efficiency was not significantly reduced. These results reveal that the water containing ultra fine bubbles enabled efficient extraction of various components such as azadirachtins A and B, nimbin, and salannin from the plant material even when the organic solvent was not used or was used in a reduced amount.
The liquid extract of each Example contained each active component described above and did not contain the solvent or contained the solvent in a reduced amount, thereby highly maintaining potency and the like and having excellent safety. Therefore, such a liquid extract can be used as, for example, an inactivator of dead mites and the like (allergy-causing substance) an pest repellent, and a bactericide.

### (Example 4)

Dried bonito flakes (Marutomo Co., Ltd.) were cut into a size of about 1 cm × 1 cm per side and to 1 g of the obtained material, 20 mL of the ion-exchanged water containing ultra fine bubbles obtained in Preparation Example 1 was added to prepare a mixture. The mixture was sonicated at 28 kHz with an ultrasonic apparatus (US CLEANER, type USD-4R, manufactured by As One Corporation) at 20°C for 6 minutes to perform extraction treatment. Then, the residue was removed by filtration with a filter paper to afford a filtrate (also called liquid extract). The obtained liquid extract was further filtered with a 0.22 µm sterile syringe filter (Millipore Millex GP PES) and the filtrate was analyzed using ultra high performance liquid chromatography (ACQULITY UPLC H-Class System (PDA), manufactured by Nihon Waters K. K.) to determine the amount of inosinic acid contained in the liquid extract. The measurement result is shown in Table 2.
The measurement condition was as follows. A phosphate buffer solution (pH 3) was used as a solvent A and acetonitrile was used as a solvent B in a gradient condition of 20-80% of the solvent B from 0 to 3 minutes, 80-100% of the solvent B from 3 to 4.5 minutes, and 100% of the solvent B from 4.5 to 7 minutes. The flow rate was 0.5 mL/min, the detection was at 254 nm, and a BEH C18 column was used.

### (Example 5)

The extraction treatment, the filtration, and the analysis of the filtrate (liquid extract) were carried out in the same manner as in Example 4 except that an ion-exchanged water containing ultra fine bubbles pre-warmed at 80°C was used and the mixture was warmed for 15 minutes in a large incubator (type CR-2200, manufactured by ESPEC) at 80°C in place of the sonication. The measurement result is shown in Table 2.

### (Comparative Example 4)

The extraction treatment, the filtration, and the analysis of the filtrate (liquid extract) were carried out in the same manner as in Example 4 except that 20 mL of ion-exchanged water without ultra fine bubbles was used in place of 20 mL of the ion-exchanged water containing ultra fine bubbles. The measurement result is shown in Table 2.

### (Comparative Example 5)

The extraction treatment, the filtration, and the analysis of the filtrate (liquid extract) were carried out in the same manner as in Example 5 except that 20 mL of ion-exchanged water without ultra fine bubbles was used in place of 20 mL of the ion-exchanged water containing ultra fine bubbles. The measurement result is shown in Table 2.

**[Table 2]**

| | | | Example 4 | Example 5 | Comparative Example 4 | Comparative Example 5 |
|---|---|---|---|---|---|---|
| Extraction condition | Extraction time | [min] | 6 | 15 | 6 | 15 |
| | Microbubbles | - | Air | Air | without | without |
| | Ultrasound frequency | [kHz] | 28 | - | 28 | - |
| | Shaking | - | Without | With | With | Without |
| | EtOH concentration | [% by weight] | 0 | 0 | 0 | 0 |
| | Extraction temperature | [°C] | 20 | 80 | 20 | 80 |
| Extraction amount | Inosinic acid | [µg/mL] | 1046.32 | 1415.63 | 521.31 | 715.93 |

The comparisons between Example 4 and Comparative Example 4 and between Example 5 and Comparative Example 5 reveal that the extraction using the water containing ultra fine bubbles greatly increased the extraction amount of inosinic acid.
The liquid extract of the Example contained of each of active components described above and did not contain solvents, thereby highly maintaining potency and the like and having excellent safety. Therefore, such liquid extract can be easily used for various applications.

### (Example 6)

To 1 g of previously pulverized coffee beans (regular coffee, middle ground, roasted, country of origin: Tanzania, Kawashima Coffee), 20 mL of the ion-exchanged water containing ultra fine bubbles obtained in Preparation Example 1 was added to prepare a mixture. The mixture was sonicated at 40 kHz with an ultrasonic apparatus (US CLEANER, type USD-4R, manufactured by As One Corporation) at 20°C for 5 minutes to perform extraction treatment. Then, the residue was removed by filtration with a filter paper to afford a filtrate (also called liquid extract). The obtained liquid extract was further filtered with a 0.22 µm sterile syringe filter (Millipore Millex GP PES) and the filtrate was analyzed using ultra high performance liquid chromatography (ACQULITY UPLC H-Class System (PDA), manufactured by Nihon Waters K. K.) to determine the amount of chlorogenic acid contained in the liquid extract. The measurement result is shown in Table 3.
The measurement condition was as follows. A phosphate buffer solution (pH 3) was used as a solvent A and acetonitrile was used as a solvent B in a gradient condition of 10-30% of the solvent B from 0 to 1 minutes, 30-50% of the solvent B from 1 to 3 minutes, and 50-100% of the solvent B from 3 to 4.5 minutes. The flow rate was 0.5 mL/min, the detection was at 330 nm, and a BEH C18 column was used.

### (Example 7)

In a coffee filter (Key Coffee Inc.), 7 g of coffee beans (regular coffee, middle ground, roasted, country of origin: Tanzania, Kawashima Coffee) were placed, and the coffee beans were dripped with 140 mL of an ion-exchanged water containing ultra fine bubbles pre-warmed at 80°C to produce liquid extract. In the same manner as in Example 6, the liquid extract was filtered and was analyzed to ultra high performance liquid chromatography to determine the amount of chlorogenic acid. The measurement result is shown in Table 3.

### (Comparative Example 6)

The extraction treatment, the filtration, and the analysis of the filtrate (liquid extract) were carried out in the same manner as in Example 6 except that 20 mL of ion-exchanged water without ultra fine bubbles was used in place of 20 mL of the ion-exchanged water containing ultra fine bubbles. The measurement result is shown in Table 3.

### (Comparative Example 7)

The extraction treatment and the analysis of the liquid extract were carried out in the same manner as in Example 7 except that 140 mL of ion-exchanged water without ultra fine bubbles was used in place of 140 mL of the ion-exchanged water containing ultra fine bubbles. The measurement result is shown in Table 3.

**[Table 3]**

| | | | Example 6 | Example 7 | Comparative Example 6 | Comparative Example 7 |
|---|---|---|---|---|---|---|
| Extraction condition | Extraction time | [min] | 5 | * | 5 | * |
| | Microbubbles | - | Air | Air | Without | Without |
| | Ultrasound frequency | [kHz] | 40 | - | 40 | - |
| | Shaking | - | Without | Without | Without | Without |
| | EtOH concentration | [% by weight] | 0 | 0 | 0 | 0 |
| | Extraction temperature | [°C] | 20 | 80 | 20 | 80 |
| Extraction amount | Chlorogenic acid | [µg/mL] | 126.96 | 96.09 | 95.05 | 80.76 |

| | | | | | | |
|---|---|---|---|---|---|---|
| *: Time required for dripping | | | | | | |

The comparisons between Example 6 and Comparative Example 6 and between Example 7 and Comparative Example 7 reveal that the extraction with the water containing ultra fine bubbles increased the extraction amount of chlorogenic acid.
The liquid extract of each Example contained each of the active components described above and did not contain a solvent, thereby highly maintaining potency and the like and having excellent safety. Therefore, such liquid extract can be easily used for various applications.

### (Example 8)

Leaves of red perilla (Shizen Kenko Co.) were pulverized with a mixer and to 1 g of the obtained pulverized material, 20 mL of the ion-exchanged water containing ultra fine bubbles obtained in Preparation Example 1 was added to prepare a mixture. The mixture was sonicated at 28 kHz with an ultrasonic apparatus (US CLEANER, type USD-4R, manufactured by As One Corporation) at 20°C for 15 minutes to perform extraction treatment. Then, the residue was removed by filtration with a filter paper to produce a filtrate (also called liquid extract). The obtained liquid extract was further filtered with a 0.22 µm sterile syringe filter (Millipore Millex GP PES) and the filtrate analyzed using ultra high performance liquid chromatography (ACQULITY UPLC H-Class System (PDA), manufactured by Nihon Waters K. K.) to determine the amounts of caffeic acid, ferulic acid, and rosmarinic acid contained in the liquid extract. The measurement results are shown in Table 4.
The measurement condition was as follows. A phosphate buffer solution (pH 3) was used as a solvent A, acetonitrile was used as a solvent B, and methanol was used as a solvent C in a gradient condition of 90-70% of the solvent A, 10% of the solvent B, and 0-20% of the solvent C from 0 to 2 minutes, 70-0% of the solvent A, 20-90% of the solvent B, and 10% of the solvent C from 2 to 3.5 minutes, and 0% of the solvent A, 90% of the solvent B, and 10% of the solvent C from 3.5 to 4.5 minutes. The flow rate was 0.6 mL/min, the detection was at 330 nm, and a BEH C18 column was used.

### (Comparative Example 8)

The extraction treatment, the filtration, and the analysis of the filtrate (liquid extract) were carried out in the same manner as in Example 8 except that 20 mL of ion-exchanged water without ultra fine bubbles was used in place of 20 mL of the ion-exchanged water containing ultra fine bubbles. The measurement results are shown in Table 4.

**[Table 4]**

| | | | Example 8 | Comparative Example 8 |
|---|---|---|---|---|
| Extraction condition | Extraction time | [min] | 15 | 15 |
| | Microbubbles | - | Air | without |
| | Ultrasound frequency | [kHz] | 28 | 28 |
| | Shaking | - | Without | Without |
| | EtOH concentration | [% by weight] | 0 | 0 |
| | Extraction temperature | [°C] | Room temperature | Room temperature |
| Extraction amount | Caffeic acid | [µg/mL] | 15.30 | 12.62 |
| | Ferulic acid | [µg/mL] | 50.10 | 37.33 |
| | Rosmarinic acid | [µg/mL] | 363.32 | 307.44 |

The comparison between Example 8 and Comparative Example 8 reveals that the extraction with the water containing ultra fine bubbles increased extraction amount of each of caffeic acid, ferulic acid, and rosmarinic acid.
The liquid extract of each example contained each of the active components described above and did not contain a solvent, thereby highly maintaining potency and the like and having excellent safety. Therefore, such an liquid extract can be easily used for various applications.

## Claims

1. An extraction method comprising bringing materials to be extracted into contact with water containing ultra fine bubbles for extraction treatment.

2. The extraction method according to claim 1, wherein the ultra fine bubbles have a mode diameter of 500 nm or smaller.

3. The extraction method according to claim 1 or 2, wherein 1 mL of the water contains 1,000,000 or more of the ultra fine bubbles.

4. The extraction method according to any one of claims 1 to 3, wherein the ultra fine bubbles have an electrically charged surface and the ultra fine bubbles have an absolute value of zeta potential of 5 mV or more.

5. The extraction method according to any one of claims 1 to 4, wherein the ultra fine bubbles substantially include one or more gases selected from air, oxygen, hydrogen, nitrogen, carbon dioxide, ozone, and inert gases

6. The extraction method according to any one of claims 1 to 5, wherein the water contains water-soluble solvents in an amount of 8% by weight or less.

7. The extraction method according to any one of claims 1 to 6, wherein the materials to be extracted are at least one selected from animal materials, plant materials, soils, and ores.

8. The extraction method according to any one of claims 1 to 7, further comprising applying one or more waves selected from electromagnetic waves, sound waves, and ultrasonic waves as the extraction treatment.

9. The extraction method according to any one of claims 1 to 8, wherein the ultra fine bubbles are generated using a ultra fine bubbles generating apparatus equipped with a gas-liquid shearing mixer.

10. The extraction method according to claim 9, wherein the materials to be extracted are immersed in water and the ultra fine bubbles are generated in a liquid containing the water by the ultra fine bubbles generating apparatus.

11. A liquid extract obtained by the extraction method as claimed in any one of claims 1 to 10.

12. An extract powder obtained by drying the liquid extract as claimed in claim 11.

13. A composition comprising the liquid extract as claimed in claim 11 or the extract powder as claimed in claim 12.
